# EUROPEAN PATENT APPLICATION

(11) **EP 4 624 566 A1**
(43) Date of publication of application: **01.10.2025**
(21) Application number: 23894473.0
(22) Date of filing: 14.11.2023
(51) Int. Cl.: C12N 1/20

(54) **METHOD FOR IMPROVING GASTRIC ACID/BILE ACID-RESISTING PROPERTIES OF LACTICASEIBACILLUS PARACASEI**

(30) Priority: 22.11.2022 JP 2022186124
(71) Applicant: Kabushiki Kaisha Yakult Honsha, Tokyo, 105-8660 (JP)
(72) Inventor: OANA Kosuke, Tokyo 105-8660 (JP); SHIMIZU Kensuke, Tokyo 105-8660 (JP); OISHI Kenji, Tokyo 105-8660 (JP)
(74) Representative: Wächtershäuser & Hartz Patentanwaltspartnerschaft mbB
(86) International application number: PCT/JP2023/040855
(87) International publication number: WO 2024/111464

(57) **Abstract**

A method for improving gastric acid/bile acid-resisting property of *Lacticaseibacillus paracasei* characterized by co-culturing with a lactic acid bacterium in which, when a precultured bacterial solution of the lactic acid bacterium is inoculated at 0.01 mass% in a 10 mass% skim milk culture medium and static cultured in a thermostatic chamber at 37°C, the pH is 6.0 to 6.5 after the culture period of four hours, the pH is 5.0 to 6.0 after the culture period of eight hours, the pH is 4.5 to 5.5 after the culture period of 12 hours, and the pH is 4.0 to 4.5 after the culture period of 24 hours, and *Lacticaseibacillus paracasei;* or a method for improving gastric acid/bile acid-resisting property of *Lacticaseibacillus paracasei* characterized by inoculating a precultured bacterial solution of *Lacticaseibacillus paracasei* in a culture medium containing skim milk at 0.1 to 20 mass% and culturing while adjusting the pH in such a manner that the pH becomes 5.5 to 6.25 after the culture period of eight hours, the pH becomes 4.25 to 5.5 after the culture period of 16 hours, and the pH becomes 4.0 to 4.75 after the culture period of 24 hours: is a novel method for improving resistance of *Lacticaseibacillus paracasei* to gastric acid or bile acid.

## Description

### Technical Field

The present invention relates to a method for improving gastric acid/bile acid-resisting property of *Lacticaseibacillus paracasei.*

### Background Art

Microorganisms which improve the balance of the intestinal microflora and which exhibit a beneficial effect on the health of the host have been attracting attention recently as probiotics. Lactic acid bacteria and bifidobacteria are used as such microorganisms, and it is reported that, through intake thereof, various physiological effects such as an effect of improving constipation-diarrhea, an effect of protecting against infection or suppressing allergy through improvement of immune function and an effect of preventing arteriosclerosis are obtained.

Regarding the definition of probiotics, a view including not only viable cells but also dead cells has been proposed recently. However, as the mechanism of action of probiotics, it is considered that beneficial microorganisms proliferate in the intestinal microflora and produce various metabolites, thereby excluding competing harmful microorganisms or suppressing the proliferation thereof, and thus can exhibit the function thereof more effectively. Thus, it is believed to be advantageous that the probiotics are alive in the intestines. In this manner, beneficial microorganisms which are taken reach the intestines desirably in the living state, but before reaching the intestines, there are various factors which inhibit the growth of the microorganisms, such as the temperature, the pH and oxygen. In particular, digestive fluids such as gastric juice and bile contain various digestive enzymes in addition to gastric acid and bile acid, and exposure to the digestive fluids causes serious damages such as death or, even if they survive, loss of colony-forming activity (growth potential).

Therefore, techniques for protecting beneficial microorganisms from digestive juices and improving the viability in the gastrointestinal tract have been examined. For example, a method for enhancing gastric acid/bile acid-resisting property of a microorganism itself by disrupting hrcA gene of the microorganism (PTL 1), a method for improving the viability by encapsulating a lactic acid bacterium in enteric-coated capsules and avoiding direct contact with gastric juice (PTL 2) and the like are disclosed. However, the former requires a complicated genetic engineering method, and the latter has restrictions on the dosage form. Thus, the techniques cannot be considered to be versatile. On the other hand, as a simpler method, it is reported that, when *Lactobacillus lactis* is cultured in a culture medium to which Tween 80 has been added excessively, the bile acid-resisting property thereof improves (NPL 1). When the present applicant applied the technique to *Lacticaseibacillus paracasei,* however, improvement of bile acid-resisting property was not observed, and a decrease thereof was rather observed.

Thus, the present applicant has found that gastric acid/bile acid-resisting property is improved by culturing lactic acid bacteria including *Lacticaseibacillus paracasei* in a culture medium containing oleic acid or a salt thereof and has submitted a patent application (PTL 3).

However, the technique for improving gastric acid/bile acid-resisting property of lactic acid bacteria including *Lacticaseibacillus paracasei* is a technique related to various foods and drinks using viable cells, and it is thus obvious that various techniques are preferably available.

### Citation List

### Patent Literature

PTL 1: JP2005-160A
PTL 2: JP2014-139200A
PTL 3: WO2021/256476

### Non Patent Literature

NPL 1: Kimoto H, Ohmomo S, Okamoto T. Enhancement of bile tolerance in lactococci by Tween 80. J Appl Microbiol. (2002) 92, 41-46.

### Summary of Invention

### Technical Problem

Accordingly, the problem of the invention is to provide a novel method for improving resistance of *Lacticaseibacillus paracasei* to gastric acid or bile acid.

### Solution to Problem

As a result of extensive study to solve the problem, the present inventors have found that the problem is solved using a lactic acid bacterium which achieves pH in specific ranges after specific periods of time as a lactic acid bacterium when *Lacticaseibacillus paracasei* and a lactic acid bacterium are co-cultured and have thus completed the invention.

As a result of extensive study to solve the problem, the present inventors have also found that the problem is solved by adjusting the pH after specific periods of time in specific ranges when *Lacticaseibacillus paracasei* is cultured and have thus completed the invention.

That is, the invention is a method for improving gastric acid/bile acid-resisting property of *Lacticaseibacillus paracasei* characterized by co-culturing with
a lactic acid bacterium in which,
when a precultured bacterial solution of the lactic acid bacterium of 10⁷ to 10⁹ cfu/ml is inoculated at 0.01 mass% in a 10 mass% skim milk culture medium and static cultured in a thermostatic chamber at 37°C,
the pH is 6.0 to 6.5 after the culture period of four hours,
the pH is 5.0 to 6.0 after the culture period of eight hours,
the pH is 4.5 to 5.5 after the culture period of 12 hours, and
the pH is 4.0 to 4.5 after the culture period of 24 hours.

Moreover, the invention is a method for improving gastric acid/bile acid-resisting property of *Lacticaseibacillus paracasei* characterized by
inoculating a precultured bacterial solution of *Lacticaseibacillus paracasei* in a culture medium containing skim milk at 0.1 to 20 mass% and
culturing while adjusting the pH in such a manner that
the pH becomes 5.5 to 6.25 after the culture period of eight hours,
the pH becomes 4.25 to 5.5 after the culture period of 16 hours, and
the pH becomes 4.0 to 4.75 after the culture period of 24 hours.

Furthermore, the invention is *Lacticaseibacillus paracasei* having gastric acid/bile acid-resisting property improved by the method for improving gastric acid/bile acid-resisting property.

### Advantageous Effects of Invention

According to the invention, resistance of *Lacticaseibacillus paracasei* to gastric acid or bile acid can be improved.

Therefore, when *Lacticaseibacillus paracasei* having improved resistance to gastric acid or bile acid obtained by the invention is used, a large amount of the viable cells can be delivered to the intestines, and various efficacies that *Lacticaseibacillus paracasei* currently has can also be obtained more strongly.

### Brief Description of Drawings

[FIG. 1] A figure showing the changes in pH after the respective culture periods in Example 1.
[FIG. 2] A figure showing the results of the continuous gastric acid/bile acid-resisting property test in Example 1.
[FIG. 3] A figure showing the viability rates of the continuous gastric acid/bile acid-resisting property test in Example 1.
[FIG. 4] A figure showing the changes in pH after the respective culture periods in Example 2.
[FIG. 5] A figure showing the results of the continuous gastric acid/bile acid-resisting property test in Example 3.
[FIG. 6] A figure showing the changes in pH after the respective culture periods in Example 4.
[FIG. 7] A figure showing the results of the continuous gastric acid/bile acid-resisting property test in Example 4.
[FIG. 8] A figure showing the viability rates of the continuous gastric acid/bile acid-resisting property test in Example 4.

### Description of Embodiments

The methods for improving gastric acid/bile acid-resisting property of *Lacticaseibacillus paracasei* of the invention (hereinafter, referred to "the improvement methods of the invention") include the two methods below.

### <Improvement Method 1 of Invention>

A method for improving gastric acid/bile acid-resisting property of *Lacticaseibacillus paracasei* characterized by co-culturing with
a lactic acid bacterium in which, when a precultured bacterial solution of the lactic acid bacterium of 10⁷ to 10⁹ cfu/ml is inoculated at 0.01 mass% in a 10 mass% skim milk culture medium and static cultured in a thermostatic chamber at 37°C,
the pH is 6.0 to 6.5 after the culture period of four hours,
the pH is 5.0 to 6.0 after the culture period of eight hours,
the pH is 4.5 to 5.5 after the culture period of 12 hours, and
the pH is 4.0 to 4.5 after the culture period of 24 hours.

### <Improvement Method 2 of Invention>

A method for improving gastric acid/bile acid-resisting property of *Lacticaseibacillus paracasei* characterized by
inoculating a precultured bacterial solution of *Lacticaseibacillus paracasei* in a culture medium containing skim milk at 0.1 to 20 mass% and
culturing while adjusting the pH in such a manner that
the pH becomes 5.5 to 6.25 after the culture period of eight hours,
the pH becomes 4.25 to 5.5 after the culture period of 16 hours, and
the pH becomes 4.0 to 4.75 after the culture period of 24 hours.

First, the improvement method 1 of the invention is explained. Regarding the lactic acid bacterium used in the improvement method 1 of the invention, for example, when a precultured bacterial solution of the lactic acid bacterium is inoculated at 0.01 mass% in a 10 mass% skim milk culture medium and static cultured in a thermostatic chamber at 37°C, the pH is 6.0 to 6.5 after the culture period of four hours, the pH is 5.0 to 6.0, preferably 5.4 to 5.9, after the culture period of eight hours, the pH is 4.5 to 5.5, preferably 4.6 to 5.2, after the culture period of 12 hours, and the pH is 4.0 to 4.5 after the culture period of 24 hours. The ranges of pH after the respective culture periods can be appropriately combined.

The skim milk culture medium contains 10 mass% skim milk, may also contain, for example, a component which is used for a general lactic acid bacterium medium and may contain a vitamin such as vitamin A, B vitamins, vitamin C and vitamin E, a salt such as calcium and magnesium, a nitrogen source such as meat extract, peptone, yeast extract and amino acids, a carbon source such as glucose, xylose, fructose and maltose or similar.

Moreover, the precultured bacterial solution of the lactic acid bacterium of 10⁷ to 10⁹ cfu/ml may be prepared by inoculating a cryopreserved lactic acid bacterium in a skim milk culture medium and static culturing in a thermostatic chamber at a temperature suitable for the growth of the lactic acid bacterium (preferably at 30 to 37°C) for 24 hours. The thermostatic chamber used here is not particularly limited, and for example, an incubator, a thermostatic bath may be used. Static here means placing still without stirring. The pH may be measured during the culture, for example, with a pH meter at a specified time.

Examples of such a lactic acid bacterium include lactic acid bacteria belonging to the genus *Streptococcus,* the genus *Lactococcus,* the genus *Lacticaseibacillus,* the genus *Lactiplantibacillus,* the genus *Latilactobacillus,* the genus *Ligilactobacillus,* the genus *Limosilactobacillus,* the genus *Lentilactobacill us,* and the genus *Levilactobacillus.* Of the lactic acid bacteria, lactic acid bacteria belonging to the genus *Streptococcus* and those belonging to the genus *Lactococcus* are preferable. Of the lactic acid bacteria belonging to the genus Streptococcus and those belonging to the genus Lactococcus, *Streptococcus thermophilus* and *Lactococcus lactis* are preferable, and Streptococcus thermophilus YIT 2001, Streptococcus thermophilus YIT 2021 and Lactococcus lactis YIT 2027 are more preferable. One, two or more strains of lactic acid bacteria can be used.

Of the lactic acid bacteria listed above, *Streptococcus thermophilus* YIT 2001 is deposited for an international deposit as Streptococcus thermophilus YIT 2001 (FERM BP-7538, accession date of April 5, 2001) at International Patent Organism Depositary, National Institute of Technology and Evaluation (#120, 2-5-8 Kazusakamatari, Kisarazu-shi, Chiba 2920818, Japan), which is an international depositary authority under the Budapest Treaty.

Moreover, Streptococcus thermophilus YIT 2021 is deposited for an international deposit as Streptococcus thermophilus YIT 2021 (FERM BP-7537, accession date: April 5, 2001) at International Patent Organism Depositary, National Institute of Technology and Evaluation above.

Furthermore, *Lactococcus lactis* YIT 2027 is deposited for an international deposit as *Lactococcus lactis* YIT 2027 (FERM BP-6224, accession date: January 16, 1998) at International Patent Organism Depositary, National Institute of Technology and Evaluation above.

In the improvement method 1 of the invention, the *Lacticaseibacillus paracasei* which is co-cultured with the lactic acid bacterium is not particularly limited, but examples thereof include *Lacticaseibacillus paracasei* YIT 0209 (type strain), *Lacticaseibacillus paracasei* YIT 9029, *Lacticaseibacillus paracasei* YIT 0180. One, two or more strains of the *Lacticaseibacillus paracasei* can be used. Of the *Lacticaseibacillus paracasei, Lacticaseibacillus paracasei* YIT 9029 is preferable.

*Lacticaseibacillus paracasei* YIT 9029 above is deposited for an international deposit as *Lactobacillus casei* YIT 9029 (FERM BP-1366, accession date: May 1, 1981) at International Patent Organism Depositary, National Institute of Technology and Evaluation (#120, 2-5-8 Kazusakamatari, Kisarazu-shi, Chiba 2920818, Japan), which is an international depositary authority under the Budapest Treaty. In this regard, *Lacticaseibacillus paracasei* YIT 9029 was previously classified as *Lactobacillus casei* but reclassified as *Lacticaseibacillus paracasei* after the recent reclassification of *Lactobacillus* (Zheng et al., A taxonomic note on the genus Lactobacillus: Description of 23 novel genera, emended description of the genus Lactobacillus Beijerinck 1901, and union of Lactobacillaceae and Leuconostocaceae. Int. J. Syst. Evol. Microbiol. 2020 Apr; 70(4):2782-2858 DOI 10.1099/ijsem.0.004107). In the present specification, regarding the classification of the lactic acid bacteria, the lactic acid bacteria are described according to the new classification classified based on the above document.

*Lacticaseibacillus paracasei* YIT 0209 above is a type strain and thus can be obtained from ATCC or the other sources.

In the improvement method 1 of the invention, the method for co-culturing the lactic acid bacterium above and *Lacticaseibacillus paracasei* is not particularly limited, and an example thereof is a method by inoculating the lactic acid bacterium above at 0.005 to 0.5 mass%, preferably at 0.01 to 0.1 mass%, and *Lacticaseibacillus paracasei* at 0.05 to 5 mass%, preferably at 0.1 to 1 mass%, in a culture medium and culturing these at 25 to 40°C, preferably at 30 to 37°C, for 24 to 72 hours, preferably for 40 to 50 hours. The culture may be static or shaking culture.

The culture medium is not particularly limited, and an example thereof is a skim milk culture medium. Moreover, a component which is used for a general lactic acid bacterium medium may be added to the culture medium, and a component such as a vitamin such as vitamin A, B vitamins, vitamin C and vitamin E, a salt such as calcium and magnesium, a nitrogen source such as meat extract, peptone, yeast extract and amino acids, and a carbon source such as glucose, xylose, fructose, and maltose may be contained.

By co-culturing the lactic acid bacterium and *Lacticaseibacillus paracasei* as described above, the gastric acid/bile acid-resisting property of *Lacticaseibacillus paracasei* is improved. Here, the method for observing the resistance of *Lacticaseibacillus paracasei* to gastric acid or bile acid is not particularly limited, and examples thereof include a continuous gastric acid/bile acid testing method using synthetic gastric acid or synthetic bile acid. The continuous gastric acid/bile acid testing method can be conducted, for example, by the method described in the Examples.

Moreover, the viability rate of the *Lacticaseibacillus paracasei* having improved gastric acid/bile acid-resisting property described above after the continuous gastric acid/bile acid test is, for example, three to 100 times higher than that of the case without the improvement method 1 of the invention.

Next, the improvement method 2 of the invention is explained. In the improvement method 2 of the invention, a precultured solution of *Lacticaseibacillus paracasei* is inoculated in a culture medium containing skim milk at 0.1 to 20 mass% and cultured while adjusting the pH to the ranges below after the culture periods below. The culture conditions are not particularly limited, and for example, culture may be conducted for 24 to 72 hours, preferably for 40 to 50 hours by static culture in a thermostatic chamber at 37°C.

During the culture, the method for adjusting the pH in such a manner that the pH becomes 5.5 to 6.25, preferably 5.6 to 6.2, after the culture period of eight hours, the pH becomes 4.25 to 5.5, preferably 4.4 to 5.4, after the culture period of 16 hours, and the pH becomes 4.0 to 4.75 after the culture period of 24 hours, is not particularly limited, and for example, the pH of the culture medium at a predetermined time may be adjusted with an acid such as lactic acid and hydrochloric acid or an alkali such as sodium hydrogen carbonate and sodium hydroxide. Moreover, the pH may also be adjusted by culturing with a specific microorganism or by regulating the culture temperature. The ranges of pH after the respective culture periods can be appropriately combined.

The *Lacticaseibacillus paracasei* used in the improvement method 2 of the invention may be the same as that used in the improvement method 1 of the invention.

The skim milk culture medium used in the improvement method 2 of the invention may be the same as that used in the improvement method 1 of the invention. Moreover, the thermostatic bath, the method for preparing the precultured bacterial solution, static culture and the measurement of the pH may also be the same as those used in the improvement method 1 of the invention.

By culturing *Lacticaseibacillus paracasei* while adjusting the pH as described above, the gastric acid/bile acid-resisting property of *Lacticaseibacillus paracasei* is improved. Here, the method for observing the resistance of *Lacticaseibacillus paracasei* to gastric acid or bile acid may be the same as the method used in the improvement method 1 of the invention.

In this regard, in the improvement method 2 of the invention, the same lactic acid bacterium as the lactic acid bacterium used in the improvement method 1 of the invention may be used together with *Lacticaseibacillus paracasei* during the culture, in the range which does not affect *Lacticaseibacillus paracasei.*

Moreover, the viable bacterial count and the viability rate of the *Lacticaseibacillus paracasei* having improved gastric acid/bile acid-resisting property described above after the continuous gastric acid/bile acid test are, for example, three to 19 times and 10 to 70 times higher, respectively, than those of the case without the improvement method 2 of the invention. Here, the fact that the gastric acid/bile acid-resisting property improves means that the viability rate after the continuous gastric acid/bile acid test becomes three times or more.

According to the improvement methods of the invention explained above, *Lacticaseibacillus paracasei* having improved resistance to gastric acid or bile acid can be obtained.

The *Lacticaseibacillus paracasei* can be used for a food or a drink such as lactic acid bacteria beverages and fermented milk. Moreover, the *Lacticaseibacillus paracasei* can also be used for a preparation such as a pharmaceutical product and a health food.

Specifically, when the *Lacticaseibacillus paracasei* is used for a food or a drink, the culture medium containing *Lacticaseibacillus paracasei* (and the lactic acid bacterium which is co-cultured according to the need) after the culture in the improvement method of the invention may be produced in the same manner as a food or a drink using a conventionally known culture medium containing a lactic acid bacterium.

Moreover, when the *Lacticaseibacillus paracasei* is used for a preparation, the preparation may be produced from the culture medium containing *Lacticaseibacillus paracasei* (and the lactic acid bacterium which is co-cultured according to the need) after the culture in the improvement method of the invention after separation, purification, and drying according to the need in the same manner as a preparation using a conventionally known culture medium containing a lactic acid bacterium.

Such a food or drink or a preparation contains *Lacticaseibacillus paracasei* having improved gastric acid/bile acid-resisting property contained therein and thus has higher effects than those of a food or drink or a preparation containing conventional *Lacticaseibacillus paracasei.* The effects are improvement of bowel movements, activation of NK cells, improvement of sleep quality and more.

### Examples

Hereinafter, the invention is explained in detail referring to Examples of the invention, but the invention is not limited to the Examples.

### Example 1

### Co-Culture Test:

### <Preparation of Precultured Bacterial Solutions>

The precultured bacterial solutions (1) to (5) below were prepared.

(1) Preparation of Precultured Bacterial Solution of *Lacticaseibacillus paracasei* YIT 9029
   Cryopreserved *Lacticaseibacillus paracasei* YIT 9029 was inoculated at 0.1% (v/v) in a skim milk culture medium and cultured aerobically at 37°C for 48 hours.
(2) Preparation of Precultured Bacterial Solution of *Streptococcus thermophilus* YIT 2001
   Cryopreserved *Streptococcus thermophilus* YIT 2001 was inoculated at 0.1% (v/v) in a skim milk culture medium and cultured aerobically at 37°C for 24 hours.
(3) Preparation of Precultured Bacterial Solution of *Streptococcus thermophilus* YIT 2021
   Cryopreserved *Streptococcus thermophilus* YIT 2021 was inoculated at 0.1% (v/v) in a skim milk culture medium and cultured aerobically at 37°C for 24 hours.
(4) Preparation of Precultured Bacterial Solution of *Streptococcus thermophilus* YIT 2037 (Type Strain)
   Cryopreserved *Streptococcus thermophilus* YIT 2037 (type strain) was inoculated at 0.1% (v/v) in a skim milk culture medium and cultured aerobically at 37°C for 24 hours.
(5) Preparation of Precultured Bacterial Solution of *Lactococcus lactis* YIT 2027
   Cryopreserved *Lactococcus lactis* YIT 2027 was inoculated at 0.1% (v/v) in a skim milk culture medium and cultured aerobically at 30°C for 24 hours.

### <Co-Culture>

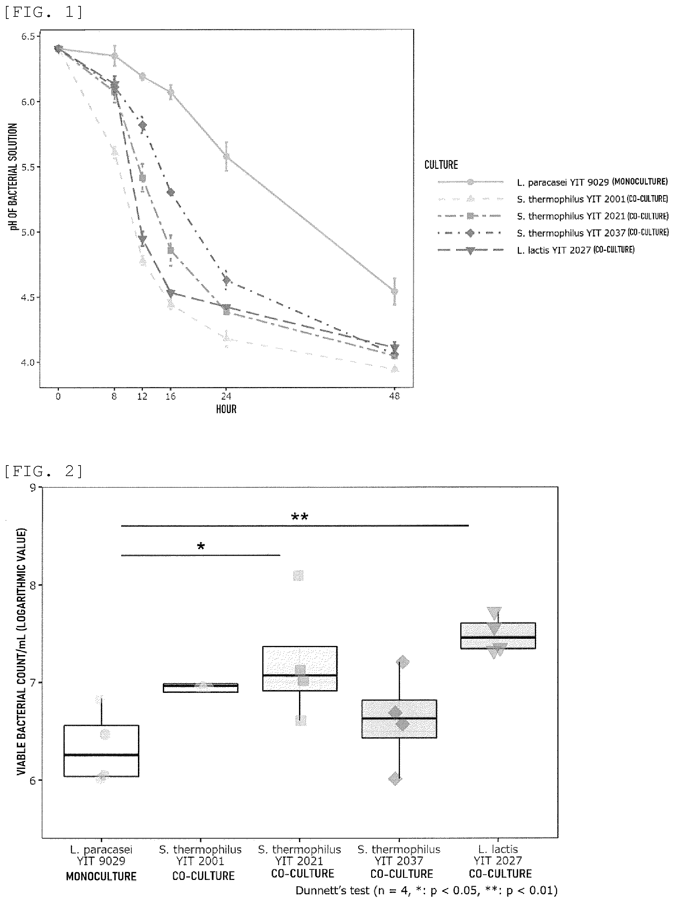

The monoculture (A) and the co-culture (B) to (E) below were conducted, and the pH values after culturing for 0, 8, 12, 16, 24 and 48 hours were measured by immersing a portable pH meter in the bacterial solutions which were stirred until uniform. The results are shown in FIG. 1.

(A) *Lacticaseibacillus paracasei* YIT 9029 was inoculated at 0.5 mass% in a 10 mass% skim milk culture medium using the precultured bacterial solution prepared in (1) and static cultured in a thermostatic chamber at 37°C.
(B) *Lacticaseibacillus paracasei* YIT 9029 and *Streptococcus thermophilus* YIT 2001 were inoculated at 0.5 mass% and 0.01 mass%, respectively, in a 10 mass% skim milk culture medium using the precultured bacterial solutions prepared in (1) and (2) and static cultured in a thermostatic chamber at 37°C.
(C) *Lacticaseibacillus paracasei* YIT 9029 and *Streptococcus thermophilus* YIT 2021 were inoculated at 0.5 mass% and 0.01 mass%, respectively, in a 10 mass% skim milk culture medium using the precultured bacterial solutions prepared in (1) and (3) and static cultured in a thermostatic chamber at 37°C.
(D) *Lacticaseibacillus paracasei* YIT 9029 and *Streptococcus thermophilus* YIT 2037 (type strain) were inoculated at 0.5 mass% and 0.01 mass%, respectively, in a 10 mass% skim milk culture medium using the precultured bacterial solutions prepared in (1) and (4) and static cultured in a thermostatic chamber at 37°C.
(E) *Lacticaseibacillus paracasei* YIT 9029 and *Lactococcus lactis* YIT 2027 were inoculated at 0.5 mass% and 0.01 mass%, respectively, in a 10 mass% skim milk culture medium using the precultured bacterial solutions prepared in (1) and (5) and static cultured in a thermostatic chamber at 37°C.

### <Continuous Gastric Acid/Bile Acid-Resisting Property Test>

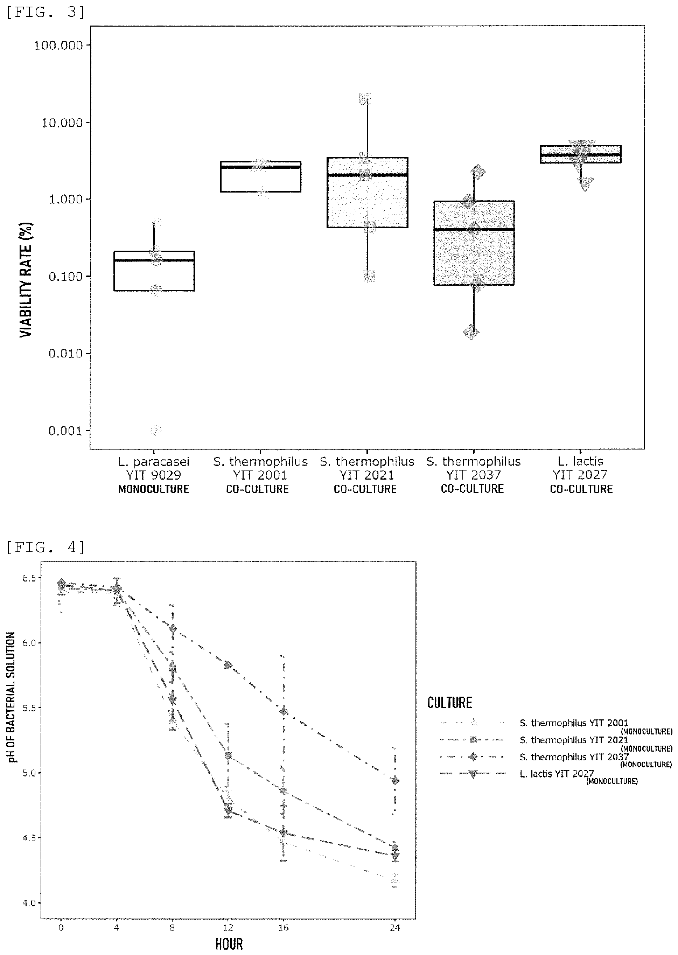

After culturing for 48 hours in the monoculture and the co-culture of (A) to (E), a continuous gastric acid/bile acid-resisting property test was conducted. The conditions of gastric acid were pH 4.0, one hour at 37°C, and the conditions of subsequent bile acid were pH 4.4, 0.3 mass% ox gall (cow's bile acid), five minutes at 37°C. The bacterial solutions which were sampled before the gastric acid exposure and after the bile acid exposure were appropriately diluted with PBS and plated on LLV agar plates. The colonies were counted after aerobically culturing for two or three days at 37°C, and the viable bacterial counts (log₁₀ cfu/ml) and the viability rates (%) of the bacterial solutions were measured. The viability rates (%) were each calculated by dividing the viable bacterial count after the gastric acid exposure and after the bile acid exposure by the viable bacterial count before the gastric acid exposure. The same test was repeated four times, and the results are shown in FIG. 2 and FIG. 3.

### <Results>

From the results, it was found that the gastric acid/bile acid-resisting property improves significantly when *Lacticaseibacillus paracasei* YIT 9029 is co-cultured with *Streptococcus thermophilus* YIT 2001, *Streptococcus thermophilus* YIT 2021 or *Lactococcus lactis* YIT 2027.

### Example 2

### Monoculture Test:

To determine what feature of a bacterial strain significantly improves gastric acid/bile acid-resisting property when the bacterial strain is co-cultured with *Lacticaseibacillus paracasei* YIT 9029, the bacterial strains used for the co-culture were cultured separately, and the pH values after culturing for 0, 4, 8, 12, 16 and 24 hours were measured with a pH meter. Here, the culture was the same as that in Example 1 except that each bacterial strain was used alone. The results are shown in FIG. 4.

From the results, it was found that those in which the pH becomes 6.0 to 6.5 after the culture period of four hours, the pH becomes 5.0 to 6.0 after the culture period of eight hours, the pH becomes 4.5 to 5.5 after the culture period of 12 hours, and the pH becomes 4.0 to 4.5 after the culture period of 24 hours, such as *Streptococcus thermophilus* YIT 2001, *Streptococcus thermophilus* YIT 2021 and *Lactococcus lactis* YIT 2027, are preferable as the lactic acid bacterium which is co-cultured with *Lacticaseibacillus paracasei* YIT 9029. On the other hand, *Streptococcus thermophilus* YIT 2037, which did not improve the gastric acid/bile acid-resisting property of *Lacticaseibacillus paracasei* YIT 9029 even when co-cultured with *Lacticaseibacillus paracasei* YIT 9029 in Example 1, did not have such a feature.

### Example 3

### Co-Culture Test:

### <Preparation of Precultured Bacterial Solutions>

The precultured bacterial solutions (6) to (10) below were prepared.

### (6) Preparation of Precultured Bacterial Solution of Lactobacillus helveticus YIT 0083 (Type Strain)

Cryopreserved *Lactobacillus helveticus* YIT 0083 (type strain) was inoculated at 0.1% (v/v) in a skim milk culture medium and cultured aerobically at 37°C for 48 hours.

### (7) Preparation of Precultured Bacterial Solution of Lactobacillus gasseri YIT 0168

Cryopreserved *Lactobacillus gasseri* YIT 0168 was inoculated at 0.1% (v/v) in a skim milk culture medium and cultured aerobically at 37°C for 48 hours. Here, *Lactobacillus gasseri* YIT 0168 is deposited for an international deposit as *Lactobacillus acidophilus* YIT 0168 according to the previous classification (FERM BP-7536, accession date: April 5, 2001) at International Patent Organism Depositary, National Institute of Technology and Evaluation above.

### (8) Preparation of Precultured Bacterial Solution of Lacticaseibacillus paracasei YIT 0180

Cryopreserved *Lacticaseibacillus paracasei* YIT 0180 was inoculated at 0.1% (v/v) in a skim milk culture medium and cultured aerobically at 37°C for 48 hours.

### (9) Preparation of Precultured Bacterial Solution of Lacticaseibacillus paracasei YIT 0209 (Type Strain)

Cryopreserved *Lacticaseibacillus paracasei* YIT 0209 (type strain) was inoculated at 0.1% (v/v) in a skim milk culture medium and cultured aerobically at 37°C for 48 hours.

### (10) Preparation of Precultured Bacterial Solution of Lacticaseibacillus paracasei YIT 9029

Cryopreserved *Lacticaseibacillus paracasei* YIT 9029 was inoculated at 0.1% (v/v) in a skim milk culture medium and cultured aerobically at 37°C for 48 hours.

### (11) Preparation of Precultured Bacterial Solution of Lactococcus lactis YIT 2027

Cryopreserved *Lactococcus lactis* YIT 2027 was inoculated at 0.1% (v/v) in a skim milk culture medium and cultured aerobically at 30°C for 24 hours.

### <Co-Culture>

The co-culture of (F) to (J) below and monoculture of each bacterial strain under the same conditions were conducted.

(F) *Lactobacillus helveticus* YIT 0083 (type strain) and *Lactococcus lactis* YIT 2027 were inoculated at 0.5 mass% and 0.01 mass%, respectively, in a 10 mass% skim milk culture medium using the precultured bacterial solutions prepared in (6) and (11) and static cultured in a thermostatic chamber at 37°C.
(G) *Lactobacillus gasseri* YIT 0168 and *Lactococcus lactis* YIT 2027 were inoculated at 0.5 mass% and 0.01 mass%, respectively, in a 10 mass% skim milk culture medium using the precultured bacterial solutions prepared in (7) and (11) and static cultured in a thermostatic chamber at 37°C.
(H) *Lacticaseibacillus paracasei* YIT 0180 and *Lactococcus lactis* YIT 2027 were inoculated at 0.5 mass% and 0.01 mass%, respectively, in a 10 mass% skim milk culture medium using the precultured bacterial solutions prepared in (8) and (11) and static cultured in a thermostatic chamber at 37°C.
(I) *Lacticaseibacillus paracasei* YIT 0209 (type strain) and *Lactococcus lactis* YIT 2027 were inoculated at 0.5 mass% and 0.01 mass%, respectively, in a 10 mass% skim milk culture medium using the precultured bacterial solutions prepared in (9) and (11) and static cultured in a thermostatic chamber at 37°C.
(J) *Lacticaseibacillus paracasei* YIT 9029 and *Lactococcus lactis* YIT 2027 were inoculated at 0.5 mass% and 0.01 mass%, respectively, in a 10 mass% skim milk culture medium using the precultured bacterial solutions prepared in (10) and (11) and static cultured in a thermostatic chamber at 37°C.

### <Continuous Gastric Acid/Bile Acid-Resisting Property Test>

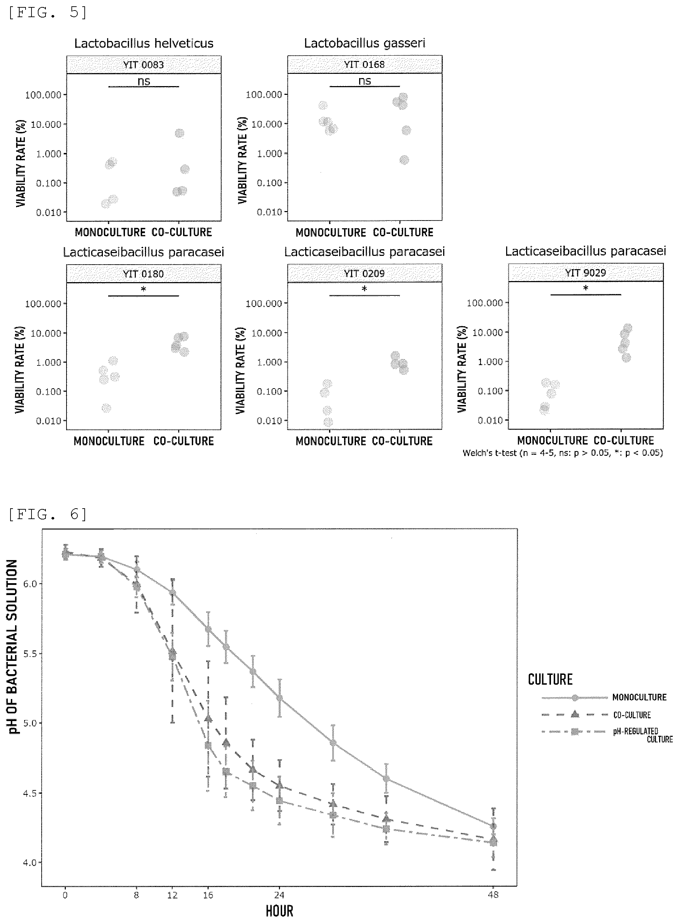

After culturing for 48 hours in the co-culture of (F) to (J) and monoculture, a continuous gastric acid/bile acid-resisting property test was conducted in the same manner as in Example 1 except for the selective media, and the viability rates (%) were calculated. The viability rates (%) were each calculated by dividing the viable bacterial count after the gastric acid exposure and after the bile acid exposure by the viable bacterial count before the gastric acid exposure. An MRS agar medium containing 0.5 µg/ml tetracycline was used as the selective medium in (F) and (G), and an MRS agar medium containing 10 µg/ml vancomycin was used in (H) to (J). The same test was repeated four or five times, and the results are shown in FIG. 5. Here, when the viability rates of *Lactobacillus helveticus* YIT 0083 (type strain) were evaluated, the conditions of bile acid were pH 4.4, 0.15 mass% ox gall, five minutes at 37°C.

### <Results>

From the results, it was found that the gastric acid/bile acid-resisting property improves significantly when *Lactococcus lactis* YIT 2027 is co-cultured with *Lacticaseibacillus paracasei* YIT 0180, *Lacticaseibacillus paracasei* YIT 0209 (type strain) or *Lacticaseibacillus paracasei* YIT 9029. On the other hand, *Lactobacillus helveticus* and *Lactobacillus gasseri,* which do not belong to *Lacticaseibacillus paracasei,* did not improve the gastric acid/bile acid-resisting property even when co-cultured with *Lactococcus lactis* YIT 2027.

### Example 4

### pH-Regulated Culture Test:

Using a culture reactor having a pH regulator (Bio Jr.8, manufactured by Biott Corporation), in the same manner as in Example 1, referring to the pH behavior during the co-culture of *Lacticaseibacillus paracasei* YIT 9029 and *Lactococcus lactis* YIT 2027, the pH during the monoculture of *Lacticaseibacillus paracasei* YIT 9029 was adjusted with lactic acid in such a manner that the pH became 5.5 to 6.25 after the culture period of eight hours, the pH became 4.25 to 5.5 after the culture period of 16 hours, and the pH became 4.0 to 4.75 after the culture period of 24 hours. The results are shown in FIG. 6.

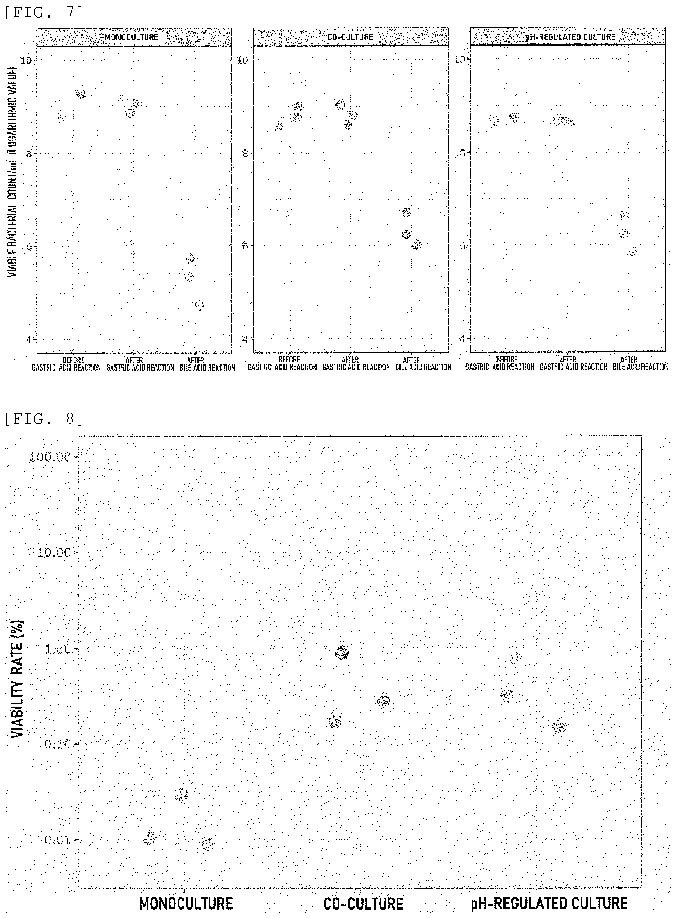

After culturing for 48 hours, a continuous gastric acid/bile acid-resisting property test was conducted in the same manner as in Example 1, and the viable bacterial counts before the gastric acid reaction, after the gastric acid reaction and after the bile acid reaction and the viability rates were calculated. The same test was repeated three times, and the results are shown in FIG. 7 and FIG. 8. Here, the conditions of bile acid were pH 4.4, 1.0 mass% ox gall, five minutes at 37°C.

From the results, it was found that the gastric acid/bile acid-resisting property of *Lacticaseibacillus paracasei* YIT 9029 improves significantly, as in the co-culture in Example 1, also when the pH is adjusted in such a manner that the pH becomes 5.5 to 6.25 after the culture period of eight hours, the pH becomes 4.25 to 5.5 after the culture period of 16 hours, and the pH becomes 4.0 to 4.75 after the culture period of 24 hours during monoculture of *Lacticaseibacillus paracasei* YIT 9029.

### Industrial Applicability

When *Lacticaseibacillus paracasei* having improved resistance to gastric acid or bile acid obtained by the invention is used, a large amount of the viable cells can be delivered to the intestines, and application to improve health becomes possible.

## Claims

1. A method for improving gastric acid/bile acid-resisting property of *Lacticaseibacillus paracasei* **characterized by** co-culturing with
a lactic acid bacterium,
wherein, when a precultured bacterial solution of the lactic acid bacterium of 10⁷ to 10⁹ cfu/ml is inoculated at 0.01mass% in a 10 mass% skim milk culture medium and static cultured in a thermostatic chamber at 37°C,
the pH is 6.0 to 6.5 after the culture period of four hours,
the pH is 5.0 to 6.0 after the culture period of eight hours,
the pH is 4.5 to 5.5 after the culture period of 12 hours, and
the pH is 4.0 to 4.5 after the culture period of 24 hours.

2. The method for improving gastric acid/bile acid-resisting property of *Lacticaseibacillus paracasei* according to claim 1, wherein the lactic acid bacterium is one or two strains selected from the group consisting of *Streptococcus thermophilus* and *Lactococcus lactis.*

3. The method for improving gastric acid/bile acid-resisting property of *Lacticaseibacillus paracasei* according to claim 1, wherein the *Lacticaseibacillus paracasei* is *Lacticaseibacillus paracasei* YIT 9029 (FERM BP-1366).

4. A method for improving gastric acid/bile acid-resisting property of *Lacticaseibacillus paracasei* **characterized by**
inoculating a precultured bacterial solution of *Lacticaseibacillus paracasei* in a culture medium containing skim milk at 0.1 to 20 mass% and
culturing while adjusting the pH in such a manner that
the pH becomes 5.5 to 6.25 after the culture period of eight hours,
the pH becomes 4.25 to 5.5 after the culture period of 16 hours, and
the pH becomes 4.0 to 4.75 after the culture period of 24 hours.

5. The method for improving gastric acid/bile acid-resisting property of *Lacticaseibacillus paracasei* according to claim 4, wherein the *Lacticaseibacillus paracasei* is *Lacticaseibacillus paracasei* YIT 9029 (FERM BP-1366).

6. *Lacticaseibacillus paracasei* having gastric acid/bile acid-resisting property improved by the method for improving gastric acid/bile acid-resisting property according to any one of claims 1 to 5.
